# FASCICULE DE BREVET EUROPEEN

(11) **EP 0 470 017 B1**
(45) Date de publication et mention de la délivrance du brevet: **14.09.1994**
(21) Numéro de dépôt: 91420277.5
(22) Date de dépôt: 25.07.1991
(51) Int. Cl.: A01N 3/00, A23B 7/01, A23B 7/06, A23L 3/01

(54) **Procédé pour stabiliser des plantes végétales**
Verfahren zur Stabilisierung von pflanzlichem Material
Method for stabilising plant material

(30) Priorité: 03.08.1990 FR 9010159
(43) Date de publication de la demande: 05.02.1992
(73) Titulaire: GATTEFOSSE S.A., 69800 Saint Priest (FR)
(72) Inventeur: Gaillard Pourrat, Aimée, F-63000 Clermont Ferrand (FR); Pourrat, Henri, F-63000 Clermont Ferrand (FR)
(74) Mandataire: Laurent, Michel

(56) Documents cités:
- FR-A- 2 553 873
- DERWENT CENTRAL PATENTS INDEX, BASIC ABSTRACTS JOURNAL, semaine 8503, classeD13, accession no. 016069/03, Derwent Publications Ltd, Londres, GB; & JP-A-59 213 356 (MITSUI TOATSU CHEM. INC.) 03-12-1984
- DERWENT CENTRAL PATENTS INDEX, BASIC ABSTRACTS JOURNAL, semaine 8705, classeD13, accession no. 033273/05, Derwent Publicatins Ltd, Londres, GB; & JP-A-61 289 855 (MIRINAGA & CO., LTD) 19-12-1986
- DERWENT CENTRAL PATENTS INDEX, BASIC ABSTRACTS JOURNAL, section C, AGDOC,semaine K09, 27 aril 1983, classe C03, accession no. 21025K/09, DerwentPublications Ltd, Londres, GB; & JP-A-58 010 502(MORINAGA & CO., LTD) 21-01-1983
- DERWENT CENTRAL PATENTS INDEX, BASIC ABSTRACTS JOURNAL, section B, semaine8514, classe B01, accession no. 083142/14, Derwent Publications Ltd, Londres,GB; & HU-A-34 219 (RICHTER GEDEONVEGY.) 28-02-1985

## Description

L'invention concerne un nouveau procédé pour stabiliser les plantes végétales (désignées ci-après "plantes"), telles que notamment les plantes à usage médical, cosmétologique, condimentaire, aromatique ou alimentaire.

Comme on le sait, les plantes présentent leurs qualités optimales au moment où elles sont cueillies. En revanche, très rapidement, ces qualités s'altèrent. Il importe donc de les "stabiliser", pour essayer de conserver le plus possible la partie essentielle et recherchée de ces propriétés.

Comme on le sait, la stabilisation des plantes fraiches est une opération qui vise à conserver à ces produits toutes leurs propriétés initiales, tant sur le plan des principes actifs thérapeutiques (alcaloïdes, oligo-éléments, vitamines, huiles essentielles, polyphénols, etc.), mais aussi d'autres propriétés telles que le goût, les couleurs et les odeurs.

Le plus généralement, pour stabiliser les plantes, on les sèche à l'air. Bien que largement répandue, cette technique présente de nombreux inconvénients liés essentiellement à la lenteur de l'opération qui entraîne des dégradations, des décolorations et des désodorisations. Tous les procédés connus à ce jour pour stabiliser les plantes visent à inactiver les enzymes qu'elles contiennent. Comme on le sait, en pratique, la dénaturation des enzymes est obtenue à une température spécifique à chaque enzyme, le plus souvent à des températures comprises entre 70 et 90°C. En raison de la mauvaise conductibilité thermique des tissus de la plante, certaines parties proches de la surface s'échauffent considérablement, alors que les zones internes voient leur température augmenter lentement. Cela est particulièrement net pour les organes épais et ligneux.

A ce jour, pour stabiliser les plantes, c'est-à-dire annihiler les enzymes, on a essentiellement utilisé des procédés qui font appel à la chaleur humide, à la vapeur d'eau, voire à la vapeur d'alcool, notamment d'éthanol. La chaleur sèche est peu utilisée, car la montée en température étant lente, les enzymes agissent malheureusement bien avant d'être détruites.

On a déjà proposé d'utiliser des courants hyper-fréquentiels sur des plantes fraiches ou fanées ou pour le séchage des graines oléagineuses, par exemple de tournesol, notamment pour améliorer la décorticabilité de ces graines. Ces techniques ne se sont pas développées, probablement en raison de leur coût élevé, car elles nécessitaient des durées de traitement extrêmement longues et ne se sont révélées utiles que pour le traitement des graines et non des plantes.

L'invention pallie ces inconvénients. Elle vise un procédé pour stabiliser les plantes végétales sans altérer leurs principes actifs thérapeutiques et tout en leur conservant leur goût et leur odeur, ce que l'on ne savait pas obtenir de manière économique et efficace jusqu'alors.

Ce procédé pour stabiliser les plantes végétales se caractérise en ce qu'il consiste :
- tout d'abord, à réhydrater les plantes fraiches pour ramener leur teneur en eau au voisinage de la teneur en eau initiale de la plante fraichement cueillie ;
- puis, à soumettre cette plante réhydratée à un traitement aux hyper-fréquences jusqu'à atteindre, dans la plante, une température au moins égale à la température de dénaturation des enzymes contenues dans cette plante.

En d'autres termes, l'invention consiste tout d'abord à réhydrater des plantes fraiches pour ramener leur teneur en eau libre (dont on sait qu'elle représente de l'ordre de 75 à 90 % de l'eau contenue dans une plante), au voisinage de leur teneur initiale, puis à soumettre cette plante superficiellement réhydratée à un traitement aux hyper-fréquences, jusqu'à atteindre une température au moins égale à la température de dénaturation des enzymes spécifiques contenues dans cette plante. Ainsi, grâce à la réhydratation superficielle, ces plantes sont fortement chargées en eau libre dont on sait qu'elle présente des pertes diélectriques très importantes, alors que les constituants essentiels du tissu (cellulose et lignine) n'ont que peu de pertes diélectriques, mais ne s'opposent pas pour autant au passage des courants hyper-fréquentiels. De la sorte, il en résulte qu'après réhydratation superficielle, lors de l'exposition ultérieure aux rayonnements hyper-fréquentiels, une montée rapide et homogène de la température, qui annihile l'action enzymatique sans modifier d'autres propriétés ou dénaturer d'autres constituants recherchés.

Le procédé selon l'invention permet d'inactiver sélectivement les enzymes contenues dans les plantes, fraiches notamment, tout en privilégiant les principes actifs thérapeutiques contenus dans ces plantes.

Comme on le sait, les "hyperfréquences" appelées aussi parfois ondes U.H.F. désignent des ondes électromagnétiques dont la longueur d'onde est de l'ordre du centimètre. Dans l'industrie, les générateurs de ces ondes sont des magnétrons, voire des klystrons.

Avantageusement, en pratique :
- on opère sur des plantes fraiches, c'est-à-dire des plantes qui ont au plus perdu le tiers de leur eau libre initiale et qui donc peuvent ainsi se réhydrater rapidement, c'est-à-dire en quelques minutes ;
- la phase de réhydratation consiste à recouvrir la plante au moins d'une fine pellicule d'eau pour, comme déjà dit, ramener la teneur en eau de la plante au voisinage de sa teneur intiale, notamment de l'ordre de 95 % de cette teneur initiale ; on a observé qu'un taux de réhydratation supérieur à 95 % est difficile à atteindre et ne permet pas d'obtenir d'amélioration notable ;
- la réhydratation s'effectue à température ambiante par immersion de la plante dans de l'eau, voire par pulvérisation, puis égouttage éventuellement suivi d'un secouage, afin d'éliminer l'eau de surface en excès, qui consommerait inutilement de l'énergie lors du traitement ultérieur aux hyperfréquences ;
- le traitement aux hyper-fréquences est associé à un chauffage infra-rouge, et peut être complété par un séchage naturel, notamment à température ambiante et en atmosphère ventilée.

La phase caractéristique de réhydratation externe superficielle permet de rétablir la circulation entre l'eau de surface et l'eau intra-cellulaire, qui est agitée par le traitement aux hyper-fréquences. Il importe donc que la teneur en eau soit suffisante pour obtenir des pertes diélectriques homogènes dans tout le volume de la plante à traiter, mais, comme déjà dit, il est inutile que cette eau de surface soit en excès.

Il en résulte que lors du traitement ultérieur aux hyperfréquences, l'échauffement interne de la plante est supérieur de quelques degrés à celui de la partie externe. Celà se traduit par une migration de l'eau des zones internes vers les zones externes d'autant plus importante que la viscosité de l'eau diminue avec l'augmentation de la température. Ce phénomène facilite le départ d'une plus grande proportion d'eau, qui favorise la dessication de la plante.

Le traitement aux hyperfréquences est effectué de manière connue, par exemple au moyen d'un four équipé de plusieurs magnétrons ou klystrons pour obtenir une meilleure répartition, une meilleure pénétration et une meilleure homogénéité des courants hyper-fréquentiels. Il importe que l'émission des ondes soit homogène. Les fréquences des magnétrons sont choisies dans les bandes autorisées pour un usage industriel. On utilise de préférence la bande B (2450 MHz). Eventuellement, on peut également utiliser la bande A, soit 915 MHz.

Dans une variante, le traitement aux hyper-fréquences est associé avec un traitement aux infra-rouges, qui a pour effet de faciliter la montée en température de l'eau. La durée du traitement varie d'une plante à l'autre, selon la nature des enzymes contenues dans la plante, selon la proportion de tissus ligneux et du volume et de la nature des plantes (feuilles, racines, etc..) à traiter. On contrôle la température par tout moyen approprié, notamment au moyen d'une sonde thermique placée au coeur du matériau à traiter. Comme déjà dit, il faut atteindre la température de dénaturation de l'effet catalytique de l'enzyme spécifique à chaque plante, que l'homme de métier connait ou peut aisément contrôler par des essais préalables simples.

Eventuellement, après ce traitement aux hyper-fréquences, la matière traitée peut subir un séchage pour ramener la teneur en eau à celle de l'eau liée. Ce séchage complémentaire peut être effectué par voie naturelle, notamment à température ambiante ou en atmosphère ventilée, voire aussi aux hyper-fréquences pour homogénéiser le séchage, notamment lorsque le surcoût de cette étape ne constitue pas un handicap économique pour les plantes concernées.

Lorsque toute l'eau libre, ou l'essentiel de celle-ci, a disparu, la plante traitée cesse d'être chauffée, de sorte que les autres constituants recherchés ne sont pas affectés.

En pratique, le procédé selon l'invention est effectué de la manière suivante.

On cueille les parties recherchées de la plante (partie aérienne, feuilles, racines..).

Les plantes à traiter encore fraiches, c'est-à-dire contenant au moins les deux tiers de leur eau initiale, sont mises à tremper par immersion à température ambiante pendant quelques minutes, puis sont égouttées. Elles sont ensuite disposées sur des supports en matériau diélectrique à faible perte. Le support avec sa charge, est alors introduit par un tapis ou tout autre moyen équivalent dans un four à hyper-fréquence qui est alors fermé. Il importe que ce four soit étanche aux radiations hyper-fréquentielles, par exemple au moyen de tresses métalliques fixées au bord ouvert de la cavité. Le four comporte des organes de sécurité et des hublots conformes aux normes exigées. Le temps d'exposition est déterminé par des essais préliminaires en fonction de la température de dénaturation des enzymes spécifiques de la plante concernée. Comme magnétrons, on utilise des magnétrons du commerce (par exemple une succession de magnétrons de 2 KW en série), notamment avec un système de refroidissement à air forcé et un système de brassage des ondes émises. Avantageusement, le four est ventilé pour évacuer la vapeur d'eau formée. On assure l'homogénéité du milieu au moyen d'un disque voilé animé d'un mouvement d'avant en arrière. Comme déjà dit, la partie inférieure du four peut éventuellement recevoir des résistances chauffantes ou des lampes à infra-rouge pour combiner un chauffage hyper-fréquentiel à un chauffage par conduction.

Les supports sont ensuite sortis du four et on achève le séchage par un procédé classique. Une partie de l'eau libre ayant été perdue pendant le traitement aux hyper-fréquences, le séchage est plus rapide, notamment en raison de la migration de l'eau des zones internes plus humides vers les zones périphériques dont la teneur en eau un peu plus faible. L'homogénéité de la teneur en eau qui en résulte favorise ainsi le départ de l'eau par capillarité.

La manière dont l'invention peut être réalisée et les avantages qui en découlent ressortiront mieux des exemples de réalisation qui suivent.

### Exemple 1 :

De manière connue, on prélève les parties aériennes (tige + feuilles) d'une passiflore fraiche (pasiflora incarnata) non fleurie.

Immédiatement après la cueillette, on dose les flavones contenues dans cette plante par la méthode classique au chlorure d'aluminium et dosage de la densité optique (DO) au spectrophotomètre dans le visible. On détermine ainsi que la plante fraichement cueillie contient environ 78 % d'eau libre par rapport au poids des tiges et feuilles analysées et comporte 3,35 % de flavones.

### Exemple 2 :

On prélève des tiges et des feuilles du même lot que l'exemple 1, et on laisse sécher ce mélange à l'air libre pendant quinze jours à 22°C dans une atmosphère à 60 % d'humidité relative, jusqu'à éliminer toute l'eau libre et à obtenir 8,5 % d'eau résiduelle.

On broie le mélange obtenu par un broyeur à couteau ou à marteau. On obtient une poudre de couleur gris-vert.

On dose les flavones dans les mêmes conditions qu'à l'exemple 1. On obtient 1,80 % de flavones.

### Exemple 3 :

Les tiges et feuilles de passiflore du même lot qu'à l'exemple 1, ont été stockées pendant trois jours à 22°C dans une humidité relative de 60 %. La quantité d'eau libre a diminué de 78 % à 55-57 %.

On immerge alors pendant quatre minutes ce matériau dans de l'eau à 22°C. On retire la plante, on la secoue pour éliminer l'excès d'eau superficiel, puis on place ces plantes sur un plateau-support de manière à obtenir une couche à peu près régulière. On introduit ensuite le plateau revêtu dans un four à micro-ondes équipé de deux magnétrons, de manière à obtenir au coeur de la couche une température voisine de 85°C. On mesure cette température par une sonde thermique.

Dès que l'on a obtenu cette température de 85°C, qui correspond aux enzymes (cellulase..) à détruire, on arrête le four, on sort le plateau, on étale le contenu que l'on sèche à l'air libre.

L'eau résiduelle contenue dans ces plantes est de 8,5 %.

Comme à l'exemple 2, on broie le mélange dans un broyeur à couteau. On obtient une poudre de couleur gris-vert, dont on dose les flavones comme précédemment. On obtient 3,35 % de flavones, c'est-à-dire qu'on a conservé exactement la même quantité de flavones que celles contenues dans la plante fraichement cueillie.

### Exemple 4 :

On répète l'exemple 3 en supprimant la phase de réhydratation.

On obtient une poudre également de couleur gris-vert comportant 2,02 % de flavones (contre 3,35 %).

### Exemple 5 :

On répète l'exemple 3 sur des feuilles de frêne (fraxinus excelsior).

L'eau résiduelle (eau liée) est de 9 %.

Les flavones mesurées comme précédemment sont de 3%, c'est-à-dire de même pourcentage que dans les feuilles de frêne fraichement cueillies.

### Exemple 6 :

On répète l'exemple 5 en supprimant l'étape de réhydratation.

On obtient une chute appréciable du taux de flavones.

### Exemple 7 :

On répète l'exemple 5 en supprimant le traitement selon l'invention, c'est-à-dire en supprimant la phase de réhydratation et le traitement aux hyperfréquences, mais en poussant le séchage naturel à 22°C 60 % HR, jusqu'à obtenir 9 % d'eau résiduelle.

On obtient alors 1,40 % de flavones.

### Exemple 8 :

On répète l'exemple 1 avec des racines de salicaire (lythrum salicaria) (et plus précisément la souche rhyzomateuse).

Ces racines fraichement cueillies présentent 6 % de tanins hydrosolubles.

Lorsque l'on sèche ces racines à l'air de manière conventionnelle, la proportion de tanins hydrosolubles tombe à 3,2 %.

Lorsque l'on fait subir à ces racines le même traitement dans les mêmes conditions qu'à l'exemple 3, on obtient 5,8 % de tanins hydrosolubles.

### Exemple 9 :

On répète l'exemple 3 sur des feuilles de sauge contenant 8,5 % d'eau résiduelle. Les feuilles traitées ont une teneur en flavones de 1,62 %.

Si on supprime la phase caractéristique de réhydratation, la teneur en eau reste la même, mais celle en flavones chute à 1,05 %. Si on supprime également le traitement aux micro-ondes, cette teneur en flavones tombe à 0,74 %.

### Exemple 10 :

On répète l'exemple 3 sur des feuilles d'estragon contenant 8,5 % d'eau. Les feuilles traitées ont une teneur en flavones de 4,30 %.

Si on supprime la phase de réhydratation, cette teneur tombe à 2,11 % et si on supprime en outre le traitement micro-ondes, cette teneur chute à 1,60 %.

Les plantes traitées de la sorte peuvent donc être ainsi conservées avec toutes leurs propriétés thérapeutiques, organoleptiques ou autres pendant plusieurs mois.

Le procédé selon l'invention permet d'obtenir une stabilisation optimale des plantes séchées ayant des propriétés analogues à celles des plantes fraiches initiales, tout en conservant leurs principes actifs, leur goût et leur odeur.

Le procédé selon l'invention permet de manière économique de récupérer l'essentiel des propriétés recherchées et optimales des plantes, sans avoir à se préoccuper de les traiter dès leur cueillette, ce qui, comme on le sait, pose des problèmes et augmente considérablement le coût.

De la sorte, ce procédé peut être utilisé avec succès pour le traitement de plantes à usages multiples, tels que alimentaire, aromatique, condimentaire, cosmétique, médicinal ou pharmaceutique.

## Revendications

1. Procédé pour stabiliser des plantes végétales, caractérisé en ce qu'il consiste :
- à réhydrater des plantes fraiches pour amener leur teneur en eau au voisinage de la teneur en eau initiale de la plante fraichement cueillie ;
- puis, à soumettre cette plante réhydratée à un traitement aux hyper-fréquences, jusqu'à atteindre dans cette plante, une température au moins égale à la température de dénaturation des enzymes spécifiques contenues dans cette plante.

2. Procédé selon la revendication 1, caractérisé en ce que la plante fraiche est réhydratée jusqu'à ce que la teneur en eau libre de la plante soit voisine de 95 % de la teneur en eau libre initiale.

3. Procédé selon la revendication 2, caractérisé en ce que la réhydratation s'effectue par immersion de la plante pendant quelques minutes dans de l'eau à température ambiante, puis égouttage pour éliminer l'eau de surface en excès.

4. Procédé selon la revendication 1, caractérisé en ce que le traitement aux hyper-fréquences est associé à un chauffage infra-rouge.

5. Procédé selon la revendication 1, caractérisé en ce que le traitement aux hyper-fréquences est effectué avec une bande de 2400 à 2500 MHz.

6. Procédé selon la revendication 1, caractérisé en ce que le traitement aux hyper-fréquences est complété par un séchage.

7. Procédé selon la revendication 6, caractérisé en ce que le séchage complémentaire est un séchage naturel effectué à température ambiante sous atmosphère ventilée.

## Claims

1. Process for stabilising vegetable plants, characterised in that it consists in:
- rehydrating the fresh plants in order to bring their water content close to the initial water content of the freshly harvested plant;
- then subjecting this rehydrated plant to an ultra-high frequency treatment until achieving in the plant a temperature at least equal to the denaturation temperature of the specific enzymes contained in this plant.

2. Process according to Claim 1, characterised in that the fresh plant is rehydrated until the free water content of the plant is close to 95% of the initial free water content.

3. Process according to Claim 2, characterised in that the rehydratation is carried out by immersing the plant for a few minutes in water at room temperature and then draining it in order to eliminate the excess surface water.

4. Process according to Claim 1, characterised in that the ultrahigh frequency treatment is combined with infrared heating.

5. Process according to Claim 1, characterised in that the ultrahigh frequency treatment is carried out within a band of 2400 to 2500 MHz.

6. Process according to Claim 1, characterised in that the ultrahigh frequency treatment is completed by drying.

7. Process according to Claim 6, characterised in that the additionnal drying is natural drying carried out at room temperature in a ventilated atmosphere.

## Patentansprüche

1. Verfahren zur Stabilisierung von Pflanzen, gekennzeichnet durch die folgenden Schritte:
- Rehydrieren der frischen Pflanzen, um ihren Wassergehalt in den Bereich des Anfangs-Wassergehaltes der frisch gepflückten Pflanze zu bringen, und anschließend
- Unterziehen dieser rehydrierten Pflanze einer Behandlung mit Ultra-Hochfrequenzen, bis in dieser Pflanze eine Temperatur erreicht wird, die mindestens gleich der Temperatur der Denaturierung der spezifischen, in dieser Pflanze enthaltenen Enzyme ist.

2. Verfahren nach Anspruch 1, dadurch gekennzeichnet, daß die frische Pflanze rehydriert wird, bis der Gehalt an freiem Wasser der Pflanze im Bereich von 95 % des Anfangsgehaltes an freiem Wasser ist.

3. Verfahren nach Anspruch 2, dadurch gekennzeichnet, daß die Rehydration durch Eintauchen oder Wässern der Pflanze während einiger Minuten in Wasser mit Umgebungstemperatur und anschließendes Abtropfen, um überschüssiges Wasser auf der Oberfläche zu entfernen, erfolgt.

4. Verfahren nach Anspruch 1, dadurch gekennzeichnet, daß die Behandlung mit Ultra-Hochfrequenzen mit einer Infrarot-Erhitzung verbunden ist.

5. Verfahren nach Anspruch 1, dadurch gekennzeichnet, daß die Behandlung mit Ultra-Hochfrequenzen in einem Bandbereich von 2400 bis 2500 MHz erfolgt.

6. Verfahren nach Anspruch 1, dadurch gekennzeichnet, daß die Behandlung mit Ultra-Hochfrequenzen durch eine Trocknung vervollständigt wird.

7. Verfahren nach Anspruch 6, dadurch gekennzeichnet, daß die ergänzende Trocknung eine natürliche Trocknung ist, die bei Umgebungstemperatur unter gelüfteter Atmosphäre erfolgt.
